# EUROPEAN PATENT APPLICATION

(11) **EP 2 505 574 A1**
(43) Date of publication of application: **03.10.2012**
(21) Application number: 10832895.6
(22) Date of filing: 22.06.2010
(51) Int. Cl.: C07C 29/15, B09B 3/00, C07C 31/04, C10J 3/00

(54) **METHANOL MANUFACTURING SYSTEM AND METHOD**

(30) Priority: 27.11.2009 JP 2009270106
(71) Applicant: Mitsubishi Heavy Industries, Ltd., Tokyo 108-8215 (JP)
(72) Inventor: MATSUMOTO, Keigo, Minato-ku, Tokyo 108-8215 (JP); FUJIMURA, Koutaro, Minato-ku, Tokyo 108-8215 (JP); KOSAKA, Kenichiro, Minato-ku, Tokyo 108-8215 (JP); HISHIDA, Masashi, Minato-ku, Tokyo 108-8215 (JP); YAMAGUCHI, Yoshiki, Minato-ku, Tokyo 108-8215 (JP); KAWAMOTO, Noboru, Minato-ku, Tokyo 108-8215 (JP); AKIBA, Toshiya, Minato-ku, Tokyo 108-8215 (JP); MIYAMOTO, Shuichi, Minato-ku, Tokyo 108-8215 (JP)
(74) Representative: Henkel, Breuer & Partner
(86) International application number: PCT/JP2010/060547
(87) International publication number: WO 2011/065046

(57) **Abstract**

Disclosed is a methanol manufacturing system provided with: a gasification furnace (20) that gasifies biomass (21) to obtain gasified biomass (24); methanol synthesis equipment (30) that synthesizes methanol (31) using refined gas (26) derived from the obtained biomass; and a gas reforming/refining device (40) that reforms and refines natural gas (41). Methanol (31) is manufactured as follows: when hydrogen-rich gas (43) derived from the natural gas is sent from the gas reforming/refining device (40) to the methanol synthesis equipment (30) and methanol is synthesized using the biomass-derived refined gas (26) and the hydrogen-rich gas (43), a control device not shown controls the process so that an index (H₂/(2CO+3CO₂) = M value) indicating the proportion of carbon sources (CO, CO₂) with respect to hydrogen is at least 1.05 after both gases (the biomass-derived refined gas (26) and the hydrogen-rich gas (43) derived from natural gas) are mixed.

## Description

### TECHNICAL FIELD

The present invention relates to a methanol production system and a methanol production method for producing methanol, which is liquid fuel, by using gasification gas derived from organic fuel such as biomass and gasification gas derived from natural gas.

### BACKGROUND ART

Biomass generally means a biological substance (for example, an agricultural product or by-product, wood, and plant) utilizable as an energy source or an industrial raw material, and is formed due to actions of solar energy, air, water, soil or the like and therefore can be endlessly recycled.

Utilizing the biomass in a form of gasification fuel enables productions of clean energy sources, such as gas and methanol for fuel. Further, biomass formed from wastes can be used and such a use results in cleaning of the environment, and newly produced biomass is grown by photosynthesis to fix CO₂. CO₂ in the air is not increased, and thus the CO₂ emission is suppressed and the use of biomass is an advantageous technique.

With respect to biomass to be supplied, preferred is one that is obtained by grinding and drying biomass which has been produced or disposed. The biomass means a biological resource (for example, an agricultural product or by-product, wood, and plant) utilizable as an energy source or an industrial raw material. For example, sweet sorghum, napier grass, or spirulina is used as the biomass (Patent documents 1 to 4 and Nonpatent literature 1).

### PRIOR ART DOCUMENTS

### PATENT DOCUMENTS

Patent document 1: Japanese Patent Application Laid-open No. 2001-240877
Patent document 2: Japanese Patent Application Laid-open No. 2001-240878
Patent document 3: Japanese Patent Application Laid-open No. H9-111254
Patent document 4: Japanese Patent Application Laid-open No. 2002-88379

### NONPATENT LITERATURE

Nonpatent literature 1: "Energy in 21st century developed by biomass", by Masayasu SAKAI, Morikita Publishing Co., Ltd., published on 28 October, 1998

### DISCLOSURE OF INVENTION

### PROBLEM TO BE SOLVED BY THE INVENTION

As for a plant in which an organic substance, such as biomass, is gasified to synthesize methanol, various proposals have been made conventionally. However, the proposed plants require more cumbersome pretreatments, as compared to apparatuses for synthesis of methanol derived from fossil fuel, and gas purification has to be performed in the plant. Therefore, conventional plants have no competitiveness in respect of the cost as a plant system for synthesizing methanol derived from biomass. Therefore, the development of a highly competitive system with cost competitiveness has been desired.

Meanwhile, in a typical methanol synthesis, gasification is performed using steam and oxygen. The reason why steam is used in the methanol synthesis is that biomass is thermally decomposed and steam needed for methanol and carbon from the organic substance are reacted with each other to increase hydrogen. The reason why oxygen is used is as follows. The methanol synthesis is performed under high pressure conditions, and therefore, when air is used in the synthesis, nitrogen contained in the air increases the required power, and further the contained nitrogen reduces the efficiency of the methanol synthesis.

Generally, CO₂ contained in biomass gasification gas cannot be used in a catalytic synthesis; however, a methanol synthesis catalyst causes a reverse reaction of the CO shift reaction {see the following formula (1)} and therefore, in addition to hydrogen (H₂) and CO, CO₂ can be used in the synthesis.

H₂+CO₂→H₂O+CO (1)

Thus, in the methanol synthesis, an ideal gas composition is such that H₂/(2CO+3CO₂)=1 or more, and the index is generally known as a so-called "M value".

For example, the system of patent document 3 has a problem in that partial combustion is caused by oxygen to generate CO₂, making it difficult to achieve the M value equal to or more than 1.

Therefore, to achieve the M value equal to or more than 1, a method of reducing CO₂ from the system can be considered. However, a CO₂ separating device needs to be separately provided and this device increases the required power, and CO₂ usable as a raw material for methanol is disadvantageously discharged from the system.

The methanol synthesis catalyst causes a reverse reaction of the CO shift reaction and therefore, even when a CO shift reaction catalyst is positioned before the synthesis, it will achieve substantially no improvement in yield of the methanol synthesis. This is because H₂ and CO₂ generated at the CO shift reaction catalyst are eventually converted to H₂O and CO, respectively, in the catalyst.

Further, patent document 4 proposes a process in which gasification is performed using steam and biomass combustion is performed outside.
In the system of patent document 4, partial combustion by oxygen is not performed, and therefore the M value equal to or more than 1 can be relatively easily achieved; however, CO₂ used for external heat is disadvantageously discharged from the system, causing a problem in that the collective methanol yield is reduced.

Thus, the gasifying system for an organic substance including biomass is generally cumbersome, and production of methanol using such a system requires a high production cost, as compared to production of methanol derived from fossil fuel.

In view of the above problems, an object of the present invention is to provide a methanol production system and a methanol production method that are advantageous in that yield of a methanol synthesis using a biomass raw material can be improved.

### MEANS FOR SOLVING PROBLEM

According to an aspect of the present invention, a methanol production system includes: a biomass gasifying furnace that gasifies biomass fed thereto to obtain biomass gasification gas; a purifying device that purifies the biomass gasification gas; a gas reforming/purifying device that reforms natural gas and purifies resultant gas to form hydrogen-rich gas; and a methanol synthesizing apparatus that synthesizes methanol using biomass-derived purified gas and natural-gas-derived hydrogen-rich gas obtained in the gas reforming/purifying device. When synthesizing methanol using the biomass-derived purified gas and the hydrogen-rich gas, methanol is produced while controlling an index specifying a ratio of a carbon source (CO, CO₂) to hydrogen {H₂/(2CO+3CO₂)= M value} using a control device so that the index becomes equal to or more than 1.05 after both (the biomass-derived purified gas and the hydrogen-rich gas) are mixed.

Advantageously, in the methanol production system, the biomass-derived purified gas is fed to the gas reforming/purifying device and reformed and purified with natural gas, and synthesis of methanol is performed using resultant mixed gas.

Advantageously, in the methanol production system, the methanol synthesizing apparatus includes: a first methanol synthesizing apparatus for biomass gasification gas; and a second methanol synthesizing apparatus for hydrogen-rich gas, the methanol production system has a hydrogen-rich off-gas feed line that feeds hydrogen-rich second off-gas from the second methanol synthesizing apparatus to a side of the first methanol synthesizing apparatus, and methanol is produced while controlling the index (M value) specifying the ratio of a carbon source (CO, CO₂) to hydrogen using a control device so that the index becomes equal to or more than 1.05.

Advantageously, in the methanol production system, the methanol synthesizing apparatus includes: a first methanol synthesizing apparatus for biomass gasification gas; and a second methanol synthesizing apparatus for hydrogen-rich gas, the methanol production system has a carbon-rich off-gas feed line that feeds carbon-rich first off-gas from the first methanol synthesizing apparatus to a side of the second methanol synthesizing apparatus, and methanol is produced while controlling the index (M value) specifying the ratio of a carbon source (CO, CO₂) to hydrogen using a control device so that the index becomes equal to or more than 1.05.

Advantageously, in the methanol production system, the methanol synthesizing apparatus includes: a first methanol synthesizing apparatus for biomass gasification gas; and a second methanol synthesizing apparatus for hydrogen-rich gas, the methanol production system has a hydrogen-rich gas feed line that feeds to a side of the purified gas a part of the hydrogen-rich gas to be fed to the second methanol synthesizing apparatus, and methanol is produced while controlling the index (M value) specifying the ratio of a carbon source (CO, CO₂) to hydrogen using a control device so that the index becomes equal to or more than 1.05.

Advantageously, in the methanol production system, the methanol synthesizing apparatus includes: a first methanol synthesizing apparatus for biomass gasification gas; and a second methanol synthesizing apparatus for hydrogen-rich gas, the methanol production system has a carbon-rich gas feed line that feeds to a side of the hydrogen-rich gas a part of the purified gas to be fed to the first methanol synthesizing apparatus, and methanol is produced while controlling the index (M value) specifying the ratio of a carbon source (CO, CO₂) to hydrogen using a control device so that the index becomes equal to or more than 1.05.

Advantageously, the methanol production system includes: a first gas composition detector that detects a gas composition of biomass gasification gas from the biomass gasifying furnace; and a second gas composition detector that detects a gas composition of hydrogen-rich gas from the gas reforming/purifying device. Methanol is produced while controlling the index (M value) specifying the ratio of a carbon source (CO, CO₂) to hydrogen using a control device, based on measurement information of the first gas composition detector and the second gas composition detector, so that the index becomes equal to or more than 1.05 after the biomass-derived purified gas and the hydrogen-rich gas are mixed.

Advantageously, the methanol production system includes a second recycle line that feeds hydrogen-lean first off-gas from the methanol synthesizing apparatus to a side of the biomass gasifying furnace.

According to another aspect of the present invention, a methanol production method includes: a step of synthesizing methanol using purified gas obtained by purifying gasification gas derived from biomass using a biomass gasifying apparatus; and a step of synthesizing methanol using hydrogen-rich gas obtained by reforming natural gas using a natural-gas reforming apparatus. When synthesizing methanol using the biomass-derived purified gas and the hydrogen-rich gas, methanol is produced while controlling an index specifying a ratio of a carbon source (CO, CO₂) to hydrogen {H₂/(2CO+3CO₂) = M value} so that the index becomes equal to or more than 1.05 after both (the biomass-derived purified gas and the hydrogen-rich gas) are mixed.

Advantageously, in the methanol production method, the methanol synthesizing apparatus is either one of a synthesizing apparatus for methanol derived from biomass and a synthesizing apparatus for methanol derived from natural gas, or is an apparatus in which these apparatuses are combined.

### EFFECT OF THE INVENTION

According to the present invention, when synthesizing methanol using the hydrogen-rich gas derived from the natural-gas reforming apparatus, which is mixed with the biomass gasification gas, the M value is controlled so that it becomes equal to or more than 1.05 after the hydrogen-rich gas and the biomass gasification gas are mixed. In this manner, the purified gas derived from a biomass gasifying apparatus and the hydrogen-rich gas derived from the natural-gas reforming apparatus complement each other to complement hydrogen with respect to the purified gas and complement the carbon source with respect to the hydrogen-rich gas, so that the yield of the methanol synthesis can be improved.

### BRIEF DESCRIPTION OF DRAWINGS

[Fig. 1] Fig. 1 is a schematic diagram of a methanol production system according to a first embodiment.
[Fig. 2] Fig. 2 is a schematic diagram of a methanol production system according to a second embodiment.
[Fig. 3] Fig. 3 is a schematic diagram of a methanol production system according to a third embodiment.
[Fig. 4] Fig. 4 is a schematic diagram of a methanol production system according to a fourth embodiment.
[Fig. 5] Fig. 5 is a schematic diagram of a methanol production system according to a fifth embodiment.
[Fig. 6] Fig. 6 is a schematic diagram of a methanol production system according to a sixth embodiment.
[Fig. 7] Fig. 7 is a schematic diagram of a methanol production system according to a seventh embodiment.
[Fig. 8] Fig. 8 is a schematic diagram of a methanol production system according to an eighth embodiment.
[Fig. 9] Fig. 9 is a schematic diagram of a methanol production system according to a ninth embodiment.
[Fig. 10] Fig. 10 depicts a relationship between an M value and a methanol calorimetric yield.

### BEST MODE(S) FOR CARRYING OUT THE INVENTION

Exemplary embodiments of the present invention will be explained below in detail with reference to the accompanying drawings. The present invention is not limited to the embodiments. In addition, constituent elements in the embodiments include those that can be easily assumed by persons skilled in the art or that are substantially equivalent.

### First embodiment

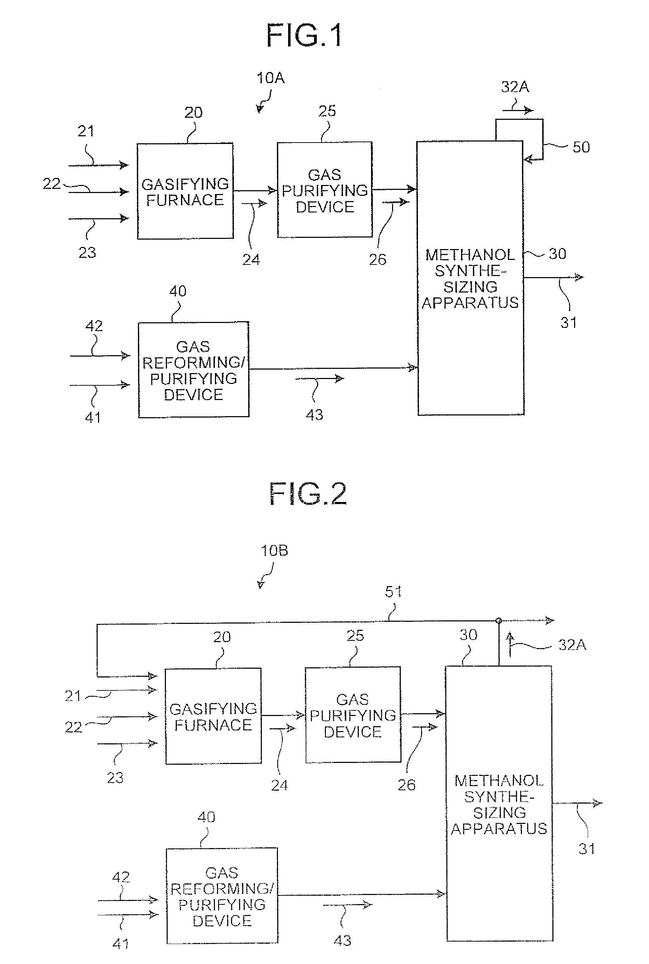

A methanol production system and a methanol production method according to a first embodiment of the present invention are explained with reference to the accompanying drawing. Fig. 1 is a schematic diagram of the methanol production system according to the present embodiment.
As shown in Fig. 1, a methanol production system 10A according to the present embodiment includes: a biomass gasifying furnace (a gasifying furnace) 20 that gasifies biomass 21 fed to the furnace to obtain biomass gasification gas 24; a gas purifying device 25 that purifies the biomass gasification gas 24; a gas reforming/purifying device 40 that reforms natural gas 41 and purifies the resultant gas to form hydrogen-rich gas; and a methanol synthesizing apparatus 30 that synthesizes methanol using biomass-derived purified gas 26 obtained in the gas purifying device 25 and natural-gas-derived hydrogen-rich gas 43 obtained in the gas reforming/purifying device 40, and when synthesizing methanol using the biomass-derived purified gas 26 and the natural-gas-derived hydrogen-rich gas 43, methanol 31 is produced while controlling an index specifying a ratio of a carbon source (CO, CO₂) to hydrogen {H₂/(2CO+3CO₂)= M value} using a control device (not shown) so that the index becomes equal to or more than 1.05 after both (the biomass-derived purified gas 26 and the natural-gas-derived hydrogen-rich gas 43) are mixed.

In the present embodiment, the biomass 21 is fed to the gasifying furnace 20, and then the biomass gasification gas 24 is purified in the gas purifying device 25 and the resultant purified gas 26 is fed to the methanol synthesizing apparatus 30 and, separately, the natural gas 41 is fed to the gas reforming/purifying device 40 to form the hydrogen-rich gas 43, and the hydrogen-rich gas 43 is fed to the methanol synthesizing apparatus 30.
In Fig. 1, reference numeral 22 denotes steam fed with the biomass 21, 23 denotes oxygen, 32A denotes first off-gas, and 42 denotes light gas. The light gas 42 is, petroleum naphtha, for example, and used as auxiliary fuel for the natural gas 41.

In the present embodiment, hydrogen-lean first off-gas 32A discharged from the methanol synthesizing apparatus 30 is recycled through a first recycle line 50 to the methanol synthesizing apparatus 30 and reused therein.

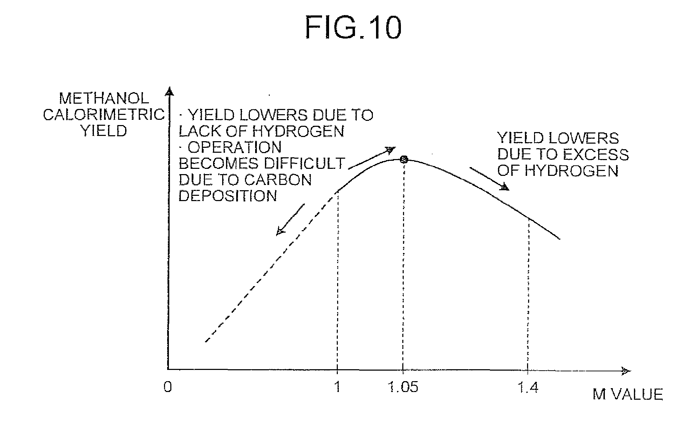

In this manner, in the present embodiment, a biomass gasifying apparatus having the biomass gasifying furnace 20 that gasifies the biomass 21 to obtain the biomass gasification gas 24 and a natural-gas reforming apparatus (a natural gas apparatus) having the gas reforming/purifying device 40 that reforms the natural gas 41 and the light gas 42 and purifies the resultant gas are used and, when synthesizing the methanol 31 using the natural-gas-derived hydrogen-rich gas 43 mixed with the biomass gasification gas 24, the M value is controlled so that it becomes equal to or more than 1.05 after the natural-gas-derived hydrogen-rich gas and the biomass-derived purified gas are mixed. Therefore, the biomass-derived purified gas 26 and the hydrogen-rich gas 43 derived from the natural-gas reforming apparatus complement each other to complement hydrogen with respect to the biomass-derived purified gas 26 and complement the carbon source with respect to the hydrogen-rich gas 43, so that the yield of synthesis of the methanol 31 can be improved.
A relationship between the M value and the methanol calorimetric yield is shown in Fig. 10. As shown in Fig. 10, the methanol calorimetric yield was improved when the M value was 1.05. When the M value exceeded 1.05, an excess of hydrogen was caused to lower the yield. It was found that, when the M value was less than 1.05, a lack of hydrogen further lowered the yield and the deposition of carbon made the operation difficult.

In the methanol synthesizing apparatus 30, gas mainly constituted by hydrogen and CO (the purified gas 26 and the hydrogen-rich gas 43) is increased in pressure and the methanol 31 is synthesized in a synthesizer filled with a catalyst under a pressure of 3 to 11 megapascals at a temperature of 180 to 300°C, and the methanol 31 is separated as a product using a gas-liquid separator provided in the synthesizer. Dimethyl ether (DME) can be synthesized from the methanol 31 by a dehydration reaction.

In this manner, the use of the biomass gasifying apparatus alone is unsatisfactory and a natural gas apparatus for fossil fuel, that is, natural gas is separately required; however, the hydrogen gas derived from biomass, carbon monoxide gas, and carbon dioxide gas can be effectively utilized and they complement each other, thereby considerably improving the yield of ethanol production.

This process is particularly effective when the methanol synthesizing apparatus is larger than the biomass gasifying apparatus.
Specifically, with respect to "production of methanol derived from a natural gas" before mixed, in the use of the apparatus for the purpose alone, for example, when the amount of natural gas used as a feedstock for the apparatus is 100 t/day, the methanol yield is 60 to 70% and the methanol yield amount is 180 t/day at an M value of 1.5.
On the other hand, with respect to "production of methanol derived from biomass", in the use of the apparatus for the purpose alone, for example, when the amount of biomass used as a feedstock for the apparatus is 100 t/day, the methanol yield is 40 to 50% and the methanol yield amount is 40 t/day.
As described above, when the apparatuses were individually operated, even the total methanol yield amount for the both apparatuses was 220 t/day.

Meanwhile, in the use of the both apparatuses in combination, with respect to "production of methanol derived from a natural gas", when the amount of natural gas used as a feedstock for the apparatus is 100 t/day, the methanol yield is also 60 to 70% and the methanol yield amount is also 180 t/day at an M value of 1.5.
However, in the use of the apparatuses in combination, with respect to "production of methanol derived from biomass", for example, when the amount of biomass used as a feedstock for the apparatus was 100 t/day, the methanol yield was 100 to 110% and the methanol yield amount was 100 t/day. The total methanol yield amount for the both apparatuses was 280 t/day.
The methanol yield amount in the production of methanol derived from biomass was 40 t/day when the biomass apparatus alone is used, whereas that was 100 t/day when the biomass apparatus and the natural gas apparatus are used in combination, which confirmed that the use of the both apparatuses in combination enabled the methanol production in an amount of 2.5 times larger than that in the use of a single biomass apparatus.

As described above, with respect to the biomass gasifying plant, the use of both of the apparatuses in combination enables synthesis of methanol in an amount almost equal to the amount of the biomass originally charged.
Further, the total methanol yield amount is 220 t/day when the apparatuses are individually used, whereas that is 280 t/day when the both apparatuses are used in combination.
The ratio of systems varies depending on operating conditions of the biomass gasifying plant and the natural-gas methanol plant; however, taking an excess of hydrogen into consideration, it is desired that the ratio for the natural-gas methanol plant is one time or more.

### Second embodiment

A methanol production system and a methanol production method according to a second embodiment of the present invention are explained with reference to the drawing. Fig. 2 is a schematic diagram of the methanol production system.
As shown in Fig. 2, a methanol production system 10B according to the present embodiment is substantially the same as the methanol production system 10A according to the first embodiment shown in Fig. 1, except that the hydrogen-lean first off-gas 32A discharged from the methanol synthesizing apparatus 30 is fed through a second recycle line 51 back to the gasifying furnace 20 that gasifies the biomass 21 and reused therein.

The off-gas 32 can be used as, for example, carrier gas for the biomass 21 and introduced to the inside of the gasifying furnace 20 with the biomass 21.

The off-gas can be used in, for example, controlling the temperature in the gasifying furnace 20 by feeding the off-gas into the gasifying furnace 20 separately from the biomass 21.

The off-gas can be used as a raw material for the methanol synthesis by causing methane or the like remaining in the first off-gas 32A to perform partial combustion.

The recycle ratio for the first off-gas 32A (the ratio of the recycled first off-gas) can be 0.1 to 0.9 and, by controlling the M value for the mixed gas of the biomass-derived purified gas 26 and the natural-gas-derived hydrogen-rich gas 43 so that the M value becomes about 1.05, the recycle ratio can be further increased, and ideally the first off-gas 32A can be rendered zero.

When a partial-combustion gasifying furnace is used as the gasifying furnace 20 to which the first off-gas 32A is fed, the off-gas 32 is introduced particularly to a portion of the furnace through which oxygen is fed to cause methane to perform combustion, making it possible to change the methane into CO and CO₂ usable for methanol.
Methane is also contained in reformed gas from the natural gas 41 although it is as small as several percents.

### Third embodiment

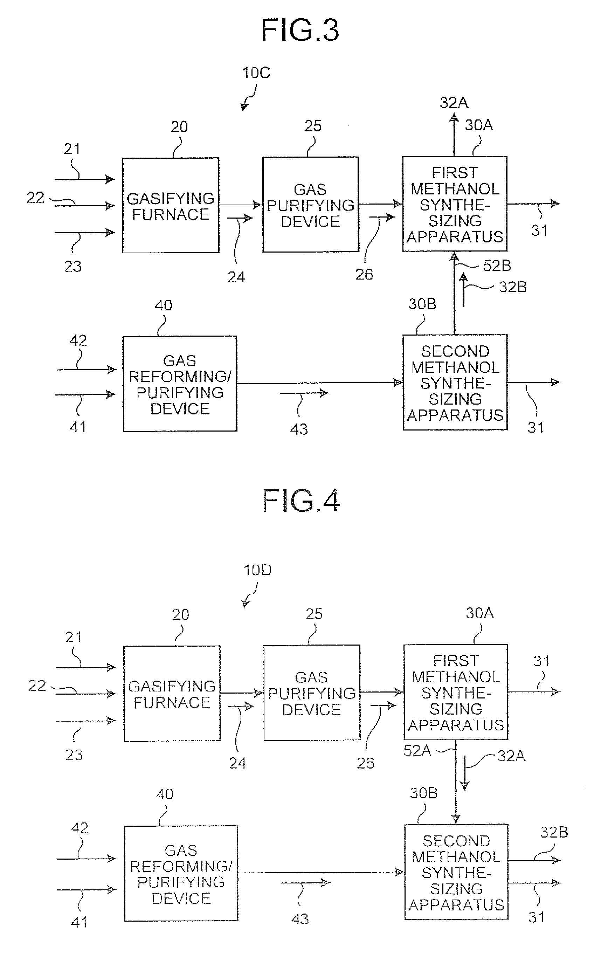

A methanol production system and a methanol production method according to a third embodiment of the present invention are explained with reference to the drawing. Fig. 3 is a schematic diagram of the methanol production system.
As shown in Fig. 3, a methanol production system 10C according to the present embodiment is substantially the same as the methanol production system 10A according to the first embodiment shown in Fig. 1, except that, instead of the methanol synthesizing apparatus 30, two separate apparatuses, which are a first methanol synthesizing apparatus 30A and a second methanol synthesizing apparatus 30B, are used.
That is, in the present embodiment, the first methanol synthesizing apparatus 30A that synthesizes methanol from the biomass-derived purified gas 26 and the second methanol synthesizing apparatus 30B that synthesizes methanol from the natural-gas-derived hydrogen-rich gas 43 are used, and further second off-gas (hydrogen-rich off-gas) 32B discharged from the second methanol synthesizing apparatus 30B is fed to the first methanol synthesizing apparatus 30A through a second off-gas feed line 52B.

As a result, when synthesizing the methanol 31 using the second off-gas 32B from the natural-gas-derived hydrogen-rich gas 43, which is mixed with the biomass-derived purified gas 26, the M value is controlled using a control device (not shown) so that the M value becomes equal to or more than 1.05 after the second off-gas and the biomass-derived purified gas are mixed, complementing hydrogen with respect to the biomass-derived purified gas 26, so that the methanol synthesis efficiency is improved.

In the present embodiment, the M value varies depending on the scale or operating conditions of the natural gas plant; however, like in the first embodiment, for obtaining gas such that the M value is about 1.05, it is desired that the scale of the natural gas feedstock (the natural gas 41) is three or more times than that of the biomass feedstock (the biomass 21).

The present embodiment is particularly preferred when an existing synthesizing apparatus for methanol derived from natural gas and a methanol synthesizing apparatus using a biomass gasifying apparatus, which is newly provided, are used in combination to produce the methanol 31.

### Fourth embodiment

A methanol production system and a methanol production method according to a fourth embodiment of the present invention are explained with reference to the drawing. Fig. 4 is a schematic diagram of the methanol production system.
As shown in Fig. 4, like the methanol production system 10C according to the third embodiment shown in Fig. 3, a methanol production system 10D according to the present embodiment includes the first methanol synthesizing apparatus 30A that synthesizes methanol from the biomass-derived purified gas 26 and the second methanol synthesizing apparatus 30B that synthesizes methanol from the hydrogen-rich gas 43 derived from the natural gas 41, and further the first (carbon-rich) off-gas 32A discharged from the first methanol synthesizing apparatus 30A is fed to the second methanol synthesizing apparatus 30B through a first off-gas feed line 52A.

In the present embodiment, when a methanol synthesizing apparatus using a biomass gasifying apparatus is provided and used in combination with the existing synthesizing apparatus for methanol derived from natural gas, an operation such that the amount of the natural gas fed is reduced (0.8 time) is possible.

### Fifth embodiment

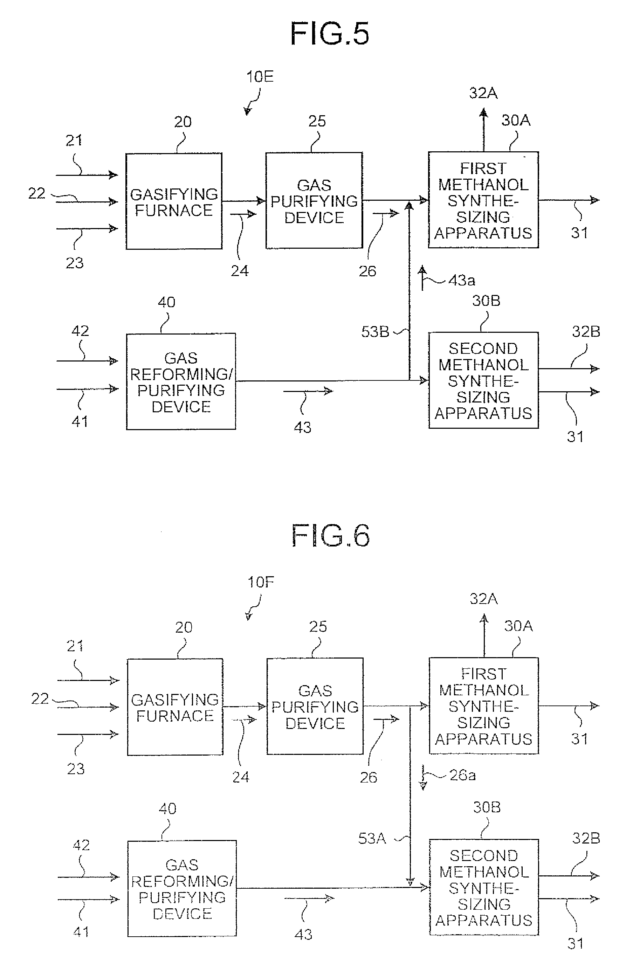

A methanol production system and a methanol production method according to a fifth embodiment of the present invention are explained with reference to the drawling. Fig. 5 is a schematic diagram of the methanol production system.
As shown in Fig. 5, like the methanol production system 10C according to the third embodiment shown in Fig. 3, a methanol production system 10E according to the present embodiment includes the first methanol synthesizing apparatus 30A that synthesizes methanol from the biomass-derived purified gas 26 and the second methanol synthesizing apparatus 30B that synthesizes methanol from the natural-gas-derived hydrogen-rich gas 43, and further a part 43a of the hydrogen-rich gas 43 before being introduced to the second methanol synthesizing apparatus 30B is fed through a second off-gas feed line 53B to a side of the purified gas 26.

When utilizing once the second off-gas 32B which has passed through the second methanol synthesizing apparatus 30B as mentioned above in the third embodiment, the pressure of gas is consequently increased twice in the two methanol synthesizing apparatuses (the pressure is increased in the second methanol synthesizing apparatus 30B and further increased in the first methanol synthesizing apparatus 30A). Therefore, with respect to the plant efficiency, the present embodiment in which the pressure is only increased once (the pressure is increased in the first methanol synthesizing apparatus 30A in this case) is more effective, making it possible to improve the plant efficiency.

### Sixth embodiment

A methanol production system and a methanol production method according to a sixth embodiment of the present invention are explained with reference to the drawing. Fig. 6 is a schematic diagram of the methanol production system.
As shown in Fig. 6, like the methanol production system 10D according to the fourth embodiment shown in Fig. 4, a methanol production system 10F according to the present embodiment includes the first methanol synthesizing apparatus 30A that synthesizes methanol from the biomass-derived purified gas 26 and the second methanol synthesizing apparatus 30B that synthesizes methanol from the natural-gas-derived hydrogen-rich gas 43, and further a part 26a of the purified gas 26 before being introduced to the first methanol synthesizing apparatus 30A is fed through a first off-gas feed line 53A to a side of the hydrogen-rich gas 43.

In the present embodiment, when utilizing once the off-gas as mentioned above in the fourth embodiment, the pressure of gas is consequently increased twice in the two methanol synthesizing apparatuses. Therefore, with respect to the plant efficiency, the present embodiment in which the pressure is only increased once is more effective, making it possible to improve the plant efficiency.

### Seventh embodiment

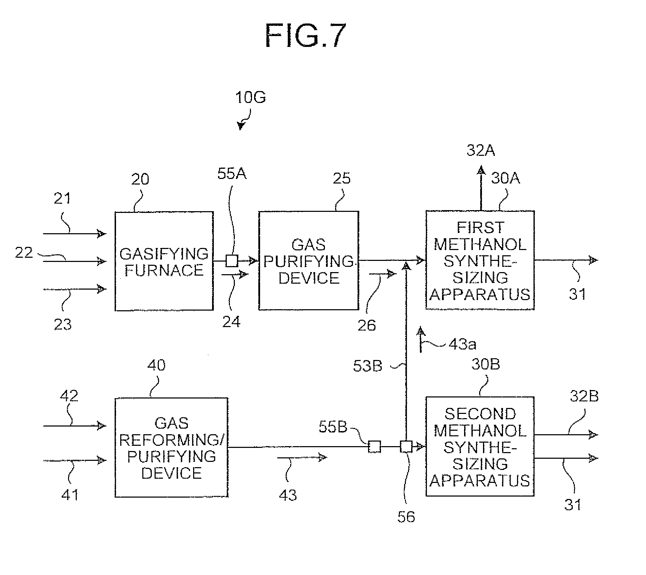

A methanol production system and a methanol production method according to a seventh embodiment of the present invention are explained with reference to the drawing. Fig. 7 is a schematic diagram of the methanol production system.
As shown in Fig. 7, a methanol production system 10G according to the present embodiment is substantially the same as the methanol production system 10E according to the third embodiment shown in Fig. 5, except that the system further includes a first gas-composition detecting device 55A that detects the gas composition of the biomass gasification gas 24 from the gasifying furnace 20, a second gas-composition detecting device 55B that detects the gas composition of the hydrogen-rich gas 43 from the gas reforming/purifying device 40, and a distribution-ratio control unit 56 that controls the amount of distribution of the hydrogen-rich gas 43.

The composition at an outlet of the gasifying furnace 20 that gasifies the biomass 21 varies depending on the composition or type of the biomass, and therefore the variation is detected by the first gas-composition detecting device 55A, and the composition of the natural-gas-derived hydrogen-rich gas 43 is detected by the second gas-composition detecting device 55B, thereby controlling the amount of distribution of the hydrogen-rich gas 43 at a gas branch using a control device (not shown) so that the M value becomes a desired value.

As for the gas-composition detecting device, a general-purpose gas detector can be used, and an analysis method by, for example, a laser Raman method is particularly preferred because the response time is short. A gas chromatography apparatus or the like can be also used.

Based on results of analysis of the gas components by the first and second gas-composition detecting devices 55A and 55B, the M value is measured and the distribution ratio is controlled so that the M value becomes about 1.05 or more after the gas mixing.

### Eighth embodiment

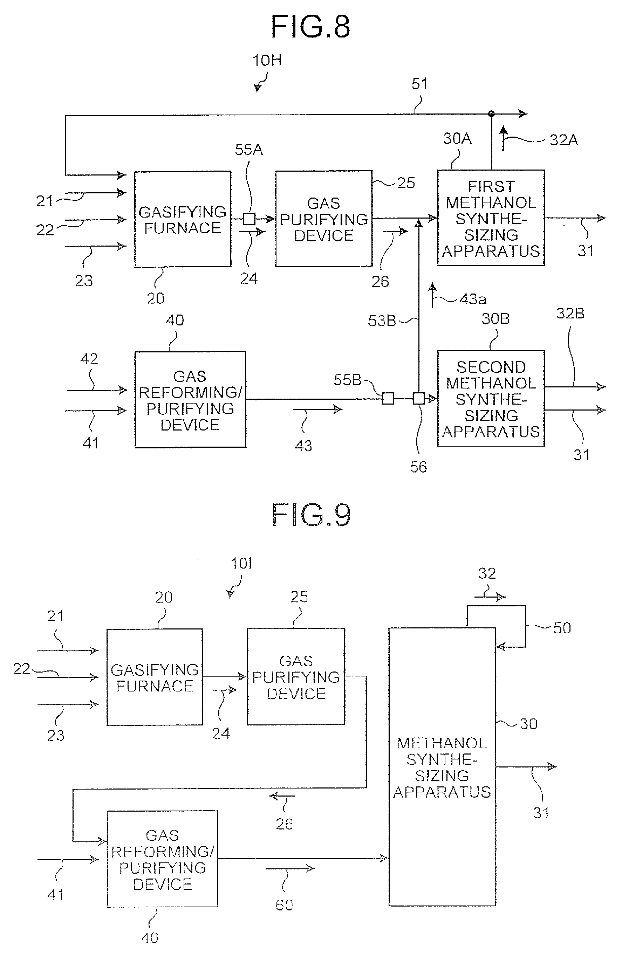

A methanol production system and a methanol production method according to an eighth embodiment of the present invention are explained with reference to the drawling. Fig. 8 is a schematic diagram of the methanol production system.
As shown in Fig. 8, a methanol production system 10H according to the present embodiment is substantially the same as the methanol production system 10G according to the seventh embodiment shown in Fig. 7, except that the first off-gas 32A discharged from the first methanol synthesizing apparatus 30A is fed through the second recycle line 51 to the gasifying furnace 20 that gasifies the biomass 21 and reused therein.
In the eighth embodiment, both the effect obtained in the seventh embodiment and the recycling effect obtained in the second embodiment can be achieved.
That is, by recycling off-gas discharged from the methanol synthesizing apparatus, not only can the remaining methane be effectively utilized, but also appropriate control of operating conditions makes it possible to eliminate the off-gas discharged from the biomass gasifying apparatus.

### Ninth embodiment

A methanol production system and a methanol production method according to a ninth embodiment of the present invention are explained with reference to the drawing. Fig. 9 is a schematic diagram of the methanol production system according to the present embodiment.
As shown in Fig. 9, a methanol production system 10I according to the present embodiment includes: the biomass gasifying furnace (the gasifying furnace) 20 that gasifies the biomass 21 fed to the furnace to obtain the biomass gasification gas 24; the gas reforming/purifying device 40 that reforms the biomass gasification gas 24 and the natural gas 41 separately fed to the device and purifies the resultant gas to form hydrogen-rich gas; and the methanol synthesizing apparatus 30 that synthesizes the methanol 31 using mixed gas 60 obtained in the gas reforming/purifying device 40, which includes the biomass-derived purified gas 26 and the hydrogen-rich gas derived from the natural gas 41 and, when synthesizing the methanol using biomass-derived purified gas 26 and the hydrogen-rich gas 43, the methanol 31 is produced while controlling an index specifying a ratio of a carbon source (CO, CO₂) to hydrogen {H₂/(2CO+3CO₂)= M value} using a control device (not shown) so that the index becomes equal to or more than 1.05 after the biomass-derived purified gas and the hydrogen-rich gas are mixed.

In the present embodiment, the purified gas 26 can be further purified in the gas reforming/purifying device 40 and therefore the purification effect is improved, thereby further improving the yield.
Accordingly, the methanol yield amount in the production of methanol derived from biomass was 40 t/day when the biomass apparatus alone was used, whereas that was 110 t/day when the biomass apparatus and the natural gas apparatus were used in combination, which confirmed that the use of both the apparatuses in combination enabled the methanol production in an amount of 2.5 or more times (10% or more larger than that in the first embodiment) than the amount in the use of a single biomass apparatus.

### INDUSTRIAL APPLICABILITY

As described above, according to the methanol production system and method of the present invention, yield of a methanol synthesis using a biomass raw material can be improved.

### EXPLANATIONS OF LETTERS OR NUMERALS

- 10A to 10I: methanol production system
- 21: biomass
- 24: biomass gasification gas
- 20: biomass gasifying furnace
- 25: gas purifying device
- 26: purified gas
- 31: methanol
- 30: methanol synthesizing apparatus
- 30A: first methanol synthesizing apparatus
- 30B: second methanol synthesizing apparatus
- 40: gas reforming/purifying device
- 41: natural gas
- 43: hydrogen-rich gas

## Claims

1. A methanol production system comprising:
a biomass gasifying furnace that gasifies biomass fed thereto to obtain biomass gasification gas;
a purifying device that purifies the biomass gasification gas;
a gas reforming/purifying device that reforms natural gas and purifies resultant gas to form hydrogen-rich gas; and
a methanol synthesizing apparatus that synthesizes methanol using biomass-derived purified gas and natural-gas-derived hydrogen-rich gas obtained in the gas reforming/purifying device, wherein
when synthesizing methanol using the biomass-derived purified gas and the hydrogen-rich gas, methanol is produced while controlling an index specifying a ratio of a carbon source (CO, CO₂) to hydrogen {H₂/(2CO+3CO₂)= M value} using a control device so that the index becomes equal to or more than 1.05 after both (the biomass-derived purified gas and the hydrogen-rich gas) are mixed.

2. The methanol production system according to claim 1, wherein the biomass-derived purified gas is fed to the gas reforming/purifying device and reformed and purified with natural gas, and synthesis of methanol is performed using resultant mixed gas.

3. The methanol production system according to claim 1, wherein
the methanol synthesizing apparatus includes:
a first methanol synthesizing apparatus for biomass gasification gas; and
a second methanol synthesizing apparatus for hydrogen-rich gas,
the methanol production system has a hydrogen-rich off-gas feed line that feeds hydrogen-rich second off-gas from the second methanol synthesizing apparatus to a side of the first methanol synthesizing apparatus, and
methanol is produced while controlling the index (M value) specifying the ratio of a carbon source (CO, CO₂) to hydrogen using a control device so that the index becomes equal to or more than 1.05.

4. The methanol production system according to claim 1, wherein
the methanol synthesizing apparatus includes:
a first methanol synthesizing apparatus for biomass gasification gas; and
a second methanol synthesizing apparatus for hydrogen-rich gas,
the methanol production system has a carbon-rich off-gas feed line that feeds carbon-rich first off-gas from the first methanol synthesizing apparatus to a side of the second methanol synthesizing apparatus, and
methanol is produced while controlling the index (M value) specifying the ratio of a carbon source (CO, CO₂) to hydrogen using a control device so that the index becomes equal to or more than 1.05.

5. The methanol production system according to claim 1, wherein
the methanol synthesizing apparatus includes:
a first methanol synthesizing apparatus for biomass gasification gas; and
a second methanol synthesizing apparatus for hydrogen-rich gas,
the methanol production system has a hydrogen-rich gas feed line that feeds to a side of the purified gas a part of the hydrogen-rich gas to be fed to the second methanol synthesizing apparatus, and
methanol is produced while controlling the index (M value) specifying the ratio of a carbon source (CO, CO₂) to hydrogen using a control device so that the index becomes equal to or more than 1.05.

6. The methanol production system according to claim 1, wherein
the methanol synthesizing apparatus includes:
a first methanol synthesizing apparatus for biomass gasification gas; and
a second methanol synthesizing apparatus for hydrogen-rich gas,
the methanol production system has a carbon-rich gas feed line that feeds to a side of the hydrogen-rich gas a part of the purified gas to be fed to the first methanol synthesizing apparatus, and
methanol is produced while controlling the index (M value) specifying the ratio of a carbon source (CO, CO₂) to hydrogen using a control device so that the index becomes equal to or more than 1.05.

7. The methanol production system according to claim 1, comprising:
a first gas composition detector that detects a gas composition of biomass gasification gas from the biomass gasifying furnace; and
a second gas composition detector that detects a gas composition of hydrogen-rich gas from the gas reforming/purifying device, wherein
methanol is produced while controlling the index (M value) specifying the ratio of a carbon source (CO, CO₂) to hydrogen using a control device, based on measurement information of the first gas composition detector and the second gas composition detector, so that the index becomes equal to or more than 1.05 after the biomass-derived purified gas and the hydrogen-rich gas are mixed.

8. The methanol production system according to claim 1, comprising a second recycle line that feeds hydrogen-lean first off-gas from the methanol synthesizing apparatus to a side of the biomass gasifying furnace.

9. A methanol production method comprising:
a step of synthesizing methanol using purified gas obtained by purifying gasification gas derived from biomass using a biomass gasifying apparatus; and
a step of synthesizing methanol using hydrogen-rich gas obtained by reforming natural gas using a natural-gas reforming apparatus, wherein
when synthesizing methanol using the biomass-derived purified gas and the hydrogen-rich gas, methanol is produced while controlling an index specifying a ratio of a carbon source (CO, CO₂) to hydrogen {H₂/(2CO+3CO₂)= M value} so that the index becomes equal to or more than 1.05 after both (the biomass-derived purified gas and the hydrogen-rich gas) are mixed.

10. The methanol production method according to claim 9, wherein the methanol synthesizing apparatus is either one
of a synthesizing apparatus for methanol derived from biomass and a synthesizing apparatus for methanol derived from natural gas, or is an apparatus in which these apparatuses are combined.
